# FASCICULE DE BREVET EUROPEEN

(11) **EP 3 259 572 B1**
(45) Date de publication et mention de la délivrance du brevet: **10.01.2024**
(21) Numéro de dépôt: 16722264.5
(22) Date de dépôt: 17.02.2016
(51) Int. Cl.: G01N 1/02, B01L 3/00

(54) **DISPOSITIF DE COLLECTION DE MATÉRIEL BIOLOGIQUE À PARTIR D'UNE TRACE BIOLOGIQUE**
VORRICHTUNG ZUR ENTNAHME VON BIOLOGISCHEM MATERIAL AUS EINER BIOLOGISCHEN SPUR
DEVICE FOR COLLECTING BIOLOGICAL MATERIAL FROM A BIOLOGICAL TRACE

(30) Priorité: 20.02.2015 FR 1500350
(43) Date de publication de la demande: 27.12.2017
(73) Titulaire: L'etat Français Représenté Par Le Ministère De L'intérieur, 92130 Issy les Mulineaux (FR)
(72) Inventeur: HUBAC, Sylvain, 95000 Cergy Pontoise (FR)
(74) Mandataire: Touroude, Magali Linda
(86) Numéro de dépôt international: PCT/FR2016/000026
(87) Numéro de publication internationale: WO 2016/132028

(56) Documents cités:
- WO-A1-88/04431
- US-A1- 2002 146 696
- US-A1- 2005 238 535
- US-A1- 2011 004 122
- US-A1- 2012 171 712
- US-A1- 2012 271 127
- US-A1- 2013 245 495
- US-A1- 2014 083 213

## Description

La présente invention concerne un dispositif de collection de matériel biologique, permettant le prélèvement de matériel biologique, son analyse et son identification de manière rapide, sans nécessité d'une étape d'extraction avant l'étape d'amplification et l'étape d'analyse et d'identification dudit matériel biologique. En particulier, ce dispositif peut être utilisé dans le domaine de la sécurité, par exemple dans le cadre d'investigations de police technique et scientifique aux fins d'analyses génétiques ou d'identification par empreintes génétiques.

A ce jour, les dispositifs de collection (appelés communément kits de prélèvement) de matériel biologique sont constituées d'écouvillons standardisés (tels que les FloqSwabs 4N6 Crime Scène Cat 3509C, Regular tip in plain tube commercialisés par la société Copan), dont des variantes sont décrites dans la demande de brevet US 2014/0083213. Ils se présentent sous la forme d'une tige, l'extrémité de laquelle est recouverte de fibres de nylon floquées, créant une zone de collection du matériel biologique de l'ordre d' 1 cm2.

On peut également aborder le document US2002146696 qui divulgue un support solide sec pour le stockage d'un échantillon de matériel génétique, ledit support comprenant une base faible, un agent chélateur, un détergent anionique, et éventuellement de l'acide urique ou un sel d'urate. Cependant, le document D8 ne divulgue pas un corps absorbant sec sous forme de fibres synthétiques floquées d'une surface comprise entre 1 et 2 mm2.

Le document US2013245495 divulgue un dispositif de collection de matériel biologique comprenant une tige présentant à une extrémité un corps absorbant sec d'une surface compris entre 2,5 mm2 et 296 mm2. Cependant, ce document ne divulgue pas un corps absorbant sec sous forme de fibres synthétiques floquées d'une surface comprise entre 1 et 2 mm2, et ne divulgue pas que ledit corps absorbant comprend des agents surfactants et dénaturants. Ce document exemplifie seulement les valeurs 7.39 mm2, 46 mm2, et 74 mm2.

Ces écouvillons sont utilisés dans les nombreux domaines qui nécessitent la collecte et l'analyse de matériel biologique tels que le domaine des analyses médicales, ou encore des analyses bactériologiques par exemple dans le domaine agro-alimentaire, cosmétique, environnementale et pharmaceutique. Ces écouvillons sont également traditionnellement utilisés par les forces de Police, par exemple sur des scènes d'infractions aux fins d'analyses génétiques ou d'identification par empreintes génétiques.

Toutefois ces écouvillons présentent de nombreux inconvénients. Tout d'abord ces écouvillons ne permettent pas de collecter de matériel biologique sous la forme de trace biologique, en particulier quand celle ci est une micro-trace, à savoir présentant une micro-surface de l'ordre de 1 mm2 ou présentant un micro-volume, de l'ordre de 1 microlitre. La demande de brevet US 2014/0083213 indique que ces écouvillons standards ne pourraient être utilisés qu'à partir d'un volume de 5 microlitres, et dans les faits, il n'est pas recommandé pour la collecte de matériel biologique d'un aussi petit volume. En outre, même sur des surfaces ou des volumes de l'ordre de 5 mm2 ou 5 microlitres, l'utilisation de tels écouvillons altèrent la trace biologique, rendant difficile voir impossible un deuxième prélèvement si besoin ou d'autres analyses, tel que l'analyse et l'identification du dessin papillaire des traces digitales dans le cas d'une trace de contact, ou encore la détermination de la nature biologique de la trace.

En outre les procédés de collecte et d'analyse et/ou d'identification de matériel biologique à l'aide de ces écouvillons nécessitent de nombreuses étapes, et en particulier au moins une étape de lyse cellulaire et d'extraction des acides nucléiques, par exemple l'ADN, avant amplification avec parfois même une étape de stabilisation de la réaction d'amplification avant de pouvoir procéder à l'analyse des produits d'amplification des acides nucléiques. Chacune des étapes d'analyse doivent être effectuées dans un environnement de laboratoire et nécessitent également des personnels techniques spécialisés, des locaux et des matériels dédiés. Les étapes d'analyse et d'identification en particulier l'étape d'extraction très contaminogène, desdits acides nucléiques, exige quant à elle un environnement protégé des contaminations. Ces contraintes excluent l'utilisation d'un analyseur standard hors du périmètre protégé d'un laboratoire conventionnel. Ces conditions sont régies par les recommandations européennes de l'ENFSI (European Network of Forensic Science Institutes) dans son programme d'assurance qualité du groupe de travail traitant de l'ADN. La durée des analyses se situe généralement aux alentours d'une dizaine d'heures pour obtenir le résultat de plusieurs dizaines de prélèvements biologiques de traces prélevées sur des scènes d'infractions (scène de crime par exemple).

Ces écouvillons nécessitent une de grande quantité de réactifs pour la lyse cellulaire et l'extraction puis pour l'amplification des acides nucléiques, lesdits réactifs étant extrêmement coûteux. En outre ces étapes doivent être réalisées dans un laboratoire d'analyse. Tout ceci rend l'utilisation de ces écouvillons et les procédés de collecte et d'analyse et/ou identification de matériel biologique mettant en oeuvre lesdits écouvillons, long, susceptibles de faire l'objet de contaminations et d'erreurs de manipulation, notamment lors du transfert du lieu de collecte du matériel biologique au laboratoire d'analyse, et bien entendu coûteux.

Ainsi il existe aujourd'hui un besoin pour des dispositifs de collection de matériel biologique permettant, avec une efficacité au moins égale quant à la collecte mais aussi l'analyse et/ou l'identification dudit matériel biologique collecté avec lesdits dispositifs,
- de sécuriser le matériel biologique collecté avec une minimisation des risques de contamination,
- de permettre la collecte de matériel biologique à l'état de micro-trace ou de micro-volume,
- de réduire la quantité de matériel biologique prélevé afin, notamment dans le cas où le matériel biologique est présent sous forme de trace, de ne pas altérer entièrement, ou a minima ladite trace biologique, notamment dans le cas des empreintes digitales ;
- d'éviter la perte d'acides nucléiques du matériel biologique collecté tel que c'est le cas lors de l'étape traditionnelle de lyse cellulaire et d'extraction ;
- de minimiser les coûts d'utilisation ;
- de minimiser le temps nécessaire entre la collecte et le résultat d'analyse et/ou l'identification du matériel biologique ;
- de permettre la collecte précise de matériel biologique dans des zones difficiles d'accès, en particulier des orifices de faible diamètre pouvant présenter des microtraces, tel que le canon d'une arme à feu, par exemple d'un diamètre de 4,5 mm, ou une cavité osseuse telle qu'une section de côte d'un être humain prélevée sur une chaîne médico-légale foraine d'identification de victimes de catastrophe.

Les développements récents en matière d'analyse ADN le plus directement possible à l'issue de la collecte du matériel biologique ne proposent pas de véritables outils « tout en un » permettant d'atteindre cet objectif et ne présentent pas non plus d'avantages par rapport aux écouvillons et méthodes d'analyses standards.

La demanderesse a mis au point les dispositifs, ainsi que le procédé de collecte, d'analyse et/ou d'identification les mettant en oeuvre, objets de la présente invention, qui permettent de résoudre l'ensemble de ce problème technique. En particulier, le dispositif selon l'invention permet de réduire l'altération de la trace biologique par une surface de prélèvement moindre, de diminuer considérablement le temps d'analyse au laboratoire, de réduire l'obstacle de la contamination par la suppression de l'étape d'extraction des acides nucléiques, en particulier de l'ADN, entre le prélèvement et le passage en analyseur et par conséquent de permettre l'utilisation d'un analyseur en série d'acides nucléiques hors du laboratoire conventionnel de génétique conventionnel permettant ainsi de disposer d'une capacité d'analyse de masse au plus près de la scène de prélèvement. Par cette méthode de collecte et d'analyse, le délai entre la collecte de la trace biologique et la détermination du profil génétique est de moins de trois heures pour les premières vingt et une traces biologiques collectées. Le système d'analyse permet ensuite de générer au moins vingt et un résultats supplémentaires toutes les vingt à vingt cinq minutes.

Le dispositif selon l'invention permet d'une part d'obtenir des résultats beaucoup plus rapidement que dans le cadre d'une analyse classique au laboratoire et d'autre part permet l'utilisation d'un analyseur ADN hors du laboratoire de génétique conventionnel soit au plus près d'une scène de crime dans un laboratoire mobile, soit au sein d'un service de police judiciaire de proximité. Les délais d'obtention des résultats sont ainsi considérablement réduits tout en conservant les avantages d'une production d'analyses en série de plusieurs dizaines de prélèvements.

Par ailleurs, l'utilisation de cet outil de collection, objet de la présente invention permet de réduire le coût de la main d'oeuvre, des équipements et des consommables de laboratoire nécessaires pour l'analyse génétique.

Le dispositif selon l'invention est particulièrement destiné à la conception et à l'industrialisation d'un dispositif universel de prélèvement de matériel biologique, très facile d'emploi, notamment par des praticiens de police technique et scientifique de premier niveau, susceptibles de l'utiliser pour la réalisation de prélèvements destinés à des laboratoires ou établissements privés ou publics.

Un premier objet de la présente invention concerne un dispositif de collection de matériel biologique, caractérisé en ce qu'il comprend une tige présentant à au moins une extrémité un corps absorbant sec sous forme de fibres synthétiques floquées d'une surface comprise entre 1 et 2 mm², ledit corps absorbant comprenant des agents surfactants et dénaturants.

Par matériel biologique, on entend désigner selon la présente invention tout matériel issu d'un être vivant, pouvant se trouver sous forme de molécules, d'organites, de fluides, de fragments cellulaires ou de cellules, et présent sur un support biologique ou non biologique.

Par support biologique, on entend un échantillon biologique, pouvant être issu de la totalité ou partie d'un organisme vivant, pouvant être décédé ou non au moment de la collecte, par exemple un être humain.
Par support non-biologique on entend désigner selon la présente invention une surface non vivante telle que celle d'un objet ou d'une structure inerte. Ces surfaces donnent des échantillons environnementaux qui, en l'absence de contact ou contamination avec du matériel biologique provenant d'un être vivant, sont exempt d'acides nucléiques contrairement aux supports biologiques. Ainsi, les échantillons obtenus à partir de supports non biologiques contenant des traces d'acides nucléiques sont le résultat d'un contact ou d'une contamination par un organisme biologique que le présent dispositif permet de collecter, d'analyser et d'identifier. Ces supports non-biologiques peuvent être de toute nature et par exemple dans le cas d'une utilisation du dispositif de collecte selon l'invention par les forces de police sur une scène d'infraction il peut s'agir typiquement d'un téléphone portable, une poignée de porte, la queue de détente ou l'intérieur du canon d'une arme, ou encore une tasse.

Le dispositif selon la présente invention est particulièrement utile pour la collecte de matériel biologique présent sur un support non-biologique, dans un environnement où de nombreux contaminants sont présents, tel que par exemple une scène d'infraction où les forces de police doivent procéder à une identification de matériel biologique, et ce d'autant plus que ledit matériel biologique est sous forme de micro-traces biologiques.

De préférence, ledit matériel biologique comprend des acides nucléiques, de préférence de l'ADN, sous une forme permettant leur amplification par PCR (Polymerase Chain Reaction) et leur analyse et/ou identification. Ledit matériel biologique est avantageusement sélectionné dans le groupe consistant en des fluides corporels physiologiques, des cellules ou suspensions cellulaires d'humains ou d'animaux, en particulier une trace de contact, du sang, de la moelle osseuse, des cellules buccales, du tissu biologique humain dégradé ou non ou des suspensions cellulaires de plantes ; des produits liquides, extraits ou suspensions de bactéries, de champignons, de plasmides, de parasites, ou de virus ; des extraits liquides ou des homogénats de tissus corporels humains ou animaux ; des milieux issus d'une synthèse d'acides nucléiques ; et des mélanges d'acides nucléiques synthétisés chimiquement ou biochimiquement. De manière préférée, ledit matériel est sélectionné dans le groupe consistant en des fluides corporels physiologiques, des cellules ou suspensions cellulaires d'humains ou d'animaux. De manière particulièrement préférée, le matériel biologique selon la présente invention est en particulier une trace de contact, du sang, de la moelle osseuse, des cellules buccales, du tissu biologique humain dégradé ou non dégradé.

Par acides nucléiques, on entend selon la présente invention l'ADN et l'ARN. De préférence, on entend désigner selon la présente invention l'ADN.

Par tige, on entend désigner de préférence selon la présente invention, une tige d'une longueur comprise entre environ 5 cm et environ 20 cm, de manière encore préférée entre environ 5 cm et environ 10 cm. De manière avantageuse la tige a un diamètre de l'ordre d'1mm. De manière avantageuse la tige est rectiligne. La tige peut être fabriquée en tout matériau adéquat selon les connaissances générales de l'Homme du métier, et peut en particulier être composée de plastique de type polypropylène. De manière préférée selon l'invention, la tige est souple.

Selon un des aspects de l'invention, une des extrémité de la tige sert de zone pour la préhension et la manipulation du dispositif, tandis ce que l'autre extrémité comprend le corps absorbant et sert pour la collecte de matériel biologique.

Selon un deuxième aspect de l'invention, la tige comprend un corps absorbant à chaque extrémité, la zone pour la préhension et le manipulation du dispositif se situant au milieu de la tige.

Par corps absorbant, on entend désigner un support solide sec composé d'un matériau absorbant, naturel ou synthétique. De préférence, ledit corps absorbant est choisi dans le groupe consistant en de la cellulose, de la nitrocellulose, du coton, des fibres synthétiques floquées, par exemple des fibres de nylon floquées, des polymères hydrophiles, de la fibre de verre, du polytétrafluoroéthylène, du polyester, du nylon, de la rayonne, du coton et une céramique poreuse. De manière encore préférée, ledit corps absorbant est choisi parmi de la cellulose, et des fibres synthétiques floquées.

Selon l'invention, ledit corps absorbant est composé de fibres synthétiques floquées, de préférence de fibres de Nylon floquées, de préférence directement sur la tige. De manière avantageuse, les fibres sont collées sur la tige. De préférence les fibres mesurent entre 1 et 1,5 mm de longueur et entre 0,01 et 0,5 mm de diamètre. Cette configuration permet d'obtenir les meilleurs résultats en terme de collecte de matériel biologique et de relargage des acides nucléiques dudit matériel biologique, associé à une simplicité de fabrication et d'utilisation.

Selon un aspect de l'invention, les fibres synthétiques sont floquées directement sur la tige, de préférence une tige souple. Selon un autre aspect de l'invention, les fibres synthétiques sont floquées sur une pastille, de préférence une pastille souple dont la souplesse permet de s'adapter à l'état de surface du support objet du prélèvement, pastille qui est fixée sur la tige. De préférence la pastille est en plastique souple, par exemple du polypropylène.

De manière avantageuse selon l'invention, les fibres synthétiques du dispositif selon l'invention permettent de piéger les acides nucléiques dudit matériel biologique, en particulier l'ADN, puis de le relarguer dans un milieu liquide, en particulier aqueux, tel qu'un mélange de réaction d'amplification par PCR d'acides nucléiques.

De manière avantageuse, lorsque le corps absorbant est composé de fibres synthétiques, de préférence en Nylon, floquées, l'amplification d'acides nucléiques par PCR peut être réalisée soit en conservant le corps absorbant dans le milieu de réaction d'amplification par PCR d'acides nucléiques et dans le thermocycleur, soit en mettant en contact le corps absorbant avec le milieu de réaction d'amplification par PCR d'acides nucléiques, en agitant puis enlevant le dit corps absorbant, permettant de relarguer lesdits acides nucléiques dans ledit milieu.

De manière avantageuse ledit corps absorbant est souple, ce qui facilite la collecte de matériel biologique. La souplesse de la tige permet de s'adapter à l'état de surface du support objet du prélèvement.

Le corps absorbant selon la présente invention présente une surface comprise entre 1 et 2 mm².

Il peut ainsi s'agir d'un disque de 1,2 mm de diamètre, correspondant à une surface 1,13 mm2.

En particulier, si la collecte est effectuée sur un matériel biologique humide, et qu'il n'y a donc pas d'étape d'humidification du matériel biologique ou du corps absorbant avant la collecte, plus la surface sera réduite, meilleurs seront les résultats d'analyse et d'identification des acides nucléiques, en particulier de l'ADN du matériel biologique prélevé.

A titre d'exemple on peut citer plusieurs type de corps absorbants selon l'invention dont la surface est de l'ordre de 1 à 2mm² à savoir :
- 1 à 2 mm² dans sa forme de fibres synthétiques floquées sur une tige ;
- environ 1,13 mm² dans sa forme de fibres synthétiques floquées sur une pastille de 1,2 mm de diamètre ;

Dans tous les cas, l'utilisation du dispositif de collection et d'analyse rapide, du fait de sa faible surface de contact et d'essuyage de la trace biologique, préserve au maximum le matériel génétique présent sur le support essuyé, qui peut ainsi faire l'objet soit d'un nouveau prélèvement, soit d'une exploitation d'une autre nature comme l'identification du dessin papillaire des traces digitales ou la détermination de la nature biologique de la trace par exemple.

De part sa superficie miniature de l'ordre de 1 à 2 mm², le corps absorbant présente à nouveau un intérêt majeur sur le plan économique car cette caractéristique permet de réaliser l'amplification d'acides nucléiques dans un volume réactionnel réduit de 50% par rapport aux préconisations du fournisseur et donc de réduire considérablement le coût des analyses d'un nombre important de prélèvements. En effet, les tests effectués avec les kits d'amplification génique Identifiler plus et Globalfiler ont permis par exemple de démontrer l'efficacité du dispositif pour un volume réactionnel de PCR de 25µl comme préconisé par le fournisseur, mais également dans un volume réactionnel réduit de moitié soit de 12,5µl.

Il a ainsi été démontré qu'une telle petite surface du corps absorbant permet de collecter une quantité suffisante d'acides nucléiques pour l'obtention d'un profil génétique.

Ledit corps absorbant est pré-traité avec des agents dénaturants et surfactants lors de sa conception permettant ainsi la lyse in situ des cellules. Il n'est donc pas nécessaire d'appliquer sur ce corps absorbant un tampon d'humidification spécifique pour la lyse des cellules en amont de la collecte.

Par agents surfactants et dénaturants on entend désigner selon la présente invention des agents permettant la lyse cellulaire, la dénaturation des protéines et l'extraction des acides nucléiques. Les agents surfactants et dénaturants peuvent être choisis parmi une liste comprenant l'acide urique ou sel d'urate, tris, SDS sel de guanidine, de préférence le thiocyanate de guanidine, Triton X100, Tween 20, CHAPS, CHAPS-O, octyl-glucoside, octyl thioglucoside et leur combinaisons.

De manière préférée selon l'invention les agents surfactants et dénaturants sont une combinaison d'acide urique ou sel d'urate, de tris, et de SDS. De préférence, le corps absorbant comprend en outre de l'EDTA.

En particulier le corps absorbant comprend un mélange par cm2 de 2 micromols d'acide urique, 8 micromols de Tris, et 1 mg de SDS. De préférence le corps absorbant comprend en outre 0,5 micromols d'EDTA.

Selon une variante de l'invention les agents surfactants et dénaturants peuvent être une combinaison de sel de guanidine, de préférence le thiocyanate de guanidine, et de Triton X100. Une quantité efficace desdits agents peut être trouvée entre 0,01M :0,01M et 0,4M :0,4M.

Des agents dénaturants et surfactants alternatifs, selon les connaissances générales de l'Homme du métier, pourront être utilisés dans le corps absorbant afin de garantir son innocuité vis à vis de la santé humaine et animale, si par exemple le dispositif devait être utilisé sur des organismes vivants, par exemple à des fins d'analyse médicale. Dans ces conditions, le corps absorbant pourra par exemple être directement appliquée dans la cavité buccale d'un individu afin d'en prélever des cellules à des fins d'analyses génétiques.

Quelque soit sa forme et sa nature, le corps absorbant comprenant ainsi des agents surfactants et dénaturants a la propriété de piéger puis de relarguer les acides nucléiques collectés ainsi rendus directement disponibles, sans délai, pour des applications de biologie moléculaire sans qu'il soit nécessaire de procéder à une étape conventionnelle d'extraction en laboratoire.

Selon un mode préféré de la présente invention, ladite extrémité présentant ledit corps absorbant du dispositif est sécable ou éjectable.

De manière particulièrement préférée, l'extrémité est sécable. Ceci peut être atteint par exemple avec un point de section sur la tige, se présentant comme une zone plus fine sur la tige, permettant de sectionner la tige par une pression, plus importante que lors de la collecte de matériel biologique, sur l'extrémité présentant le corps absorbant. De préférence ce point de section se situe à une hauteur permettant l'insertion totale du corps absorbant et de la tige sectionnée dans un récipient. De préférence ce point de section est entre 0,5 mm et 2 mm, encore préférentiellement 1,7 mm.

Selon un mode préféré de la présente invention, ledit dispositif comprend un étui hermétique dans lequel vient s'insérer tout le dispositif, ou bien une partie de la tige ainsi que l'extrémité de la tige avec le corps absorbant, à l'exclusion de la zone de préhension et de manipulation, ou bien uniquement le corps absorbant. De manière préférée, l'étui hermétique comporte un identifiant unique, par exemple par code-barres, RFID, ou NFC. De préférence l'identifiant unique selon l'invention est un code-barres.

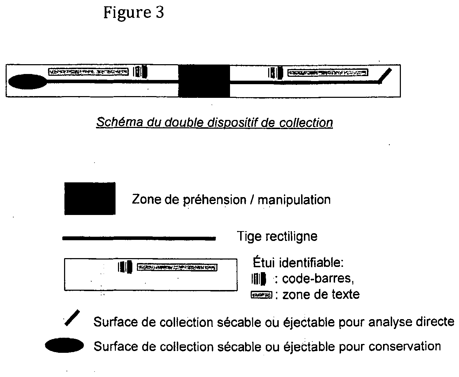

Selon une variante de l'invention illustrée par la figure 3, ce dispositif peut-être également couplé à un second dispositif de collection constitué d'une tige, de préférence d'une longueur de 5 à 10 cm environ, dont de préférence l'extrémité est sécable ou éjectable, présente un corps absorbant permettant la collection du matériel biologique. Ce second dispositif de collection peut par exemple permettre la conservation et l'extraction ultérieure des acides nucléiques aux fins d'analyses par les techniques de biologie moléculaire notamment par PCR. Dans cette configuration, les deux dispositifs sont associés mais insérés chacun dans un étui hermétique distinct, de préférence identifiable notamment par un code-barres, RFID ou NFC.

Le corps absorbant de ce second dispositif de collection peut être de taille et/ou de composition identique ou différent du corps absorbant du premier dispositif. De préférence, ledit corps absorbant du second dispositif est constitué de fibres synthétiques floquées sur la tige ou de fibres de coton enroulées autour de la tige, d'une surface comprise entre 1mm2 et 2 cm2.

De manière avantageuse selon l'invention, le dispositif de collection et en particulier son corps absorbant, subit à l'issue de la fabrication et avant utilisation un traitement par tout type de système de stérilisation à sec, notamment de type gamma irradiation, oxyde d'éthylène et plasma. Ce traitement de stérilisation permet de prévenir la contamination du dispositif de collection avant utilisation par un matériel biologique autre que celui qui sera prélevé. Ceci permet notamment au dispositif de collection de répondre aux recommandations internationales les plus exigeantes en matière de prévention de toute contamination par des acides nucléiques ne provenant pas de la trace biologique prélevée.

Le dispositif particulièrement préféré selon l'invention présente un corps absorbant solide et sec composé de fibres synthétiques floquées sur la tige, d'une surface comprise entre 1 et 2 mm2, sécable, sur une tige rectiligne de 5 à 10 cm de longueur, associé à un étui hermétique identifiable. Le corps absorbant comprend 2 micromols d'acide urique, 8 micromols de Tris, 0,5 micromols d'EDTA et 1 mg de SDS par cm2. Ce dispositif particulièrement préféré est utilisé sans humidification préalable du matériel biologique ou du corps absorbant avant collecte .

Un deuxième objet de la présente invention concerne un kit pour la collecte, l'analyse et/ou l'identification de matériel biologique comprenant un dispositif selon l'invention, deux récipients, au moins un mélange de réaction d'amplification par PCR d'acides nucléiques, un thermocycleur, un mélange de réaction de séquençage et un séquenceur.

De manière préférée selon l'invention, ledit kit peut être utilisé dans un laboratoire mobile, tel qu'un véhicule mobile, par exemple de type camionnette ou bus.

De manière préférée selon l'invention, le récipient consiste en le ou les puits d'une plaque ou une barrette, par exemple de 8 puits, 96 puits ou 384 puits et est prêt à l'emploi pour la PCR. Ainsi, de préférence, le premier récipient est pré-rempli avec le mélange de réaction d'amplification par PCR d'acides nucléiques. De manière avantageuse, ledit récipient comprend également les témoins de réaction d'amplification, pré-remplis dans le récipient et déjà ajoutés dans le mélange d'amplification prêt à l'emploi. Le second récipient est de préférence pré-rempli avec le mélange de réaction de séquençage des produits d'amplification par PCR d'acides nucléiques. De manière avantageuse ledit récipient peut également être pré-rempli, en complément du mélange de la réaction de séquençage, d'un ensemble de fragments d'ADN de référence, appelé communément échelle allélique, permettant l'identification des produits d'amplifications.

Par récipient on entend un récipient pour le corps absorbant afin d'effectuer la mise en contact dudit corps absorbant avec le mélange de réaction d'amplification par PCR d'acides nucléiques et la PCR ainsi que la mise en contact des produits d'amplification avec le mélange de réaction de séquençage. On entend par exemple désigner selon la présente invention un récipient d'une contenance de 10 à 350 microlitres, ou une combinaison de récipients d'une contenance de 10 à 200 microlitres tel qu'une microplaque PCR de 96 puits, ou de 384 puits. De préférence, le récipient est une microplaque PCR de 96 puits de 100 ou 200 microlitres.

Un troisième objet de la présente invention concerne un laboratoire mobile pour la collecte, l'analyse et/ou l'identification de matériel biologique comprenant un kit selon l'invention dans un véhicule mobile motorisé.

Ledit véhicule mobile motorisé est typiquement une camionnette ou un bus. De préférence ledit véhicule est électriquement autonome, par exemple avec un dispositif garantissant une alimentation électrique ininterrompue durant tout le processus d'amplification par PCR et le séquençage. Préférentiellement le véhicule dispose d'une liaison télématique sécurisée permettant en tout point du globe terrestre de transmettre les informations et en particulier les profils génétiques ainsi déterminés par exemple à un expert ou dans les bases de données nationales ou internationales.
Ledit laboratoire mobile a une configuration qui permet de respecter le principe de la « marche en avant » du traitement d'un échantillon, à savoir une première zone dans laquelle l'échantillon à analyser est inséré dans le laboratoire et rentre dans la zone analytique, une zone dans laquelle sont réalisées l'étape de transfert du corps absorbant dans le récipient comprenant le mélange de réaction d'amplification par PCR d'acides nucléiques, et une zone dans laquelle est réalisée la PCR dans le thermocycleur, la mise en contact des produits d'amplification avec le mélange de réaction de séquençage, et le séquençage.

Un quatrième objet de la présente invention concerne l'utilisation d'un dispositif selon l'invention pour le prélèvement de matériel biologique à partir d'une trace biologique présentant toute surface à partir d'une micro-surface de l'ordre de 1 mm2 ou présentant tout volume, à partir d'un micro-volume de l'ordre de 1 microlitre.

Selon une caractéristique de l'invention, si le prélèvement est effectué avec le dispositif directement sur un être humain, ledit être humain n'est pas vivant.

Selon une caractéristique de l'invention, si le prélèvement est effectué avec le dispositif indirectement sur un être humain, ledit être humain peut être vivant. Par indirectement, on entend ici désigner l'emploi, en amont du prélèvement, d'un outil de type stylo « auto-piqueur » utilisé par exemple pour vérifier le taux de glycémie d'un individu à partir d'une goutte de sang. L'utilisation du stylo « auto-piqueur » sur une partie du corps de l'individu vivant, en particulier un doigt de la main, provoque la génération d'une goutte de sang au niveau de zone de piqure. Cette goutte de sang est alors transférée sur une bandelette, connue de l'Homme du métier, par essuyage ou simple effet de capillarité.

Un cinquième objet de l'invention concerne un procédé de collecte de matériel biologique, caractérisé en ce qu'il comprend une étape de mise en contact du dispositif selon l'invention avec ledit matériel biologique.

Par mise en contact du dispositif avec le matériel biologique on entend désigner selon la présente invention l'essuyage du support sur lequel se trouve ledit matériel biologique à l'aide de mouvements de balayages horizontaux, circulaires ou par simples tamponnages ou capillarité en fonction de la nature de la surface à essuyer.

Lorsque le matériel biologique à collecter est humide, la collecte est effectuée directement, et lorsque le matériel biologique est sous forme sèche, ledit procédé peut comprendre une étape préalable d'humidification dudit corps absorbant du dispositif ou d'humidification dudit matériel biologique, avec de l'eau stérile et/ou apyrogène.
De manière préférée, le corps absorbant ou le matériel biologique n'est pas directement humidifié avant sa collecte. La zone sur laquelle se trouve le matériel biologique à collecter peut être alors soumise à une hygrométrie locale contrôlée qui favorise la désolidarisation du matériel biologique de son support facilitant ainsi sa collecte.

Lorsque le matériel biologique à collecter est une trace de contact sèche, invisible, telle que des cellules épithéliales présentes sur la crosse d'une arme à feu ou le manche d'un couteau, le dit procédé de collecte peut comprendre une étape préalable d'humidification du corps absorbant du dispositif ainsi que du matériel biologique avec de l'eau stérile et/ou apyrogène. Par l'intermédiaire d'un système d'aspiration de laboratoire de type à vide ou pipette à vide, connu par l'Homme du métier, la dépression d'air continue générée par le système permet d'aspirer le matériel biologique de son support qui vient alors se fixer directement sur ledit corps absorbant utilisé ici comme une membrane, un filtre du système d'aspiration.

De manière avantageuse selon l'invention, une très faible quantité d'eau stérile et/ou apyrogène, de préférence stérile et apyrogène, est nécessaire à ladite humidification, comprise entre environ 1 et environ 50 microlitres, de préférence entre environ 1 et environ 20 microlitres, de manière encore préférée entre environ 0,5 et 5 microlitres.

Un sixième objet de la présente invention concerne un procédé de collecte et d'analyse et/ou d'identification des acides nucléiques d'un matériel biologique, caractérisé en ce qu'il comprend :
1. la ou les étapes du procédé de collecte dudit matériel biologique selon l'invention,
2. une étape d'amplification des acides nucléiques, de préférence de l'ADN, dudit matériel biologique collecté sur le corps absorbant du dispositif comprenant
   ∘ la mise en contact dudit corps absorbant comprenant le matériel biologique collecté avec au moins un mélange de réaction d'amplification par PCR d'acides nucléiques, de préférence d'ADN,
   ∘ la réaction d'amplification par PCR ;
3. une étape de séquençage des acides nucléiques amplifiés à l'étape 2 comprenant la mise en contact desdits acides nucléiques avec un mélange de réaction de séquençage, le séquençage et l'analyse du résultat de séquençage.

Le procédé d'analyse selon la présente invention mettant en oeuvre le dispositif selon l'invention présente l'avantage de pouvoir passer de l'étape 1) de collecte du matériel biologique à l'étape 2) d'amplification des acides nucléiques dudit matériel biologique, sans nécessité d'une autre étape, en particulier une étape de lyse cellulaire et/ou d'extraction des acides nucléiques.

En outre, sans délai, à l'issue de la collecte, les acides nucléiques, en particulier l'ADN, piégés dans le corps absorbant du dispositif sont directement amplifiés par PCR (Polymerase Chain Reaction), sans qu'il soit nécessaire d'ajouter un additif pour stabiliser la réaction d'amplification, aux fins par exemple de détermination de profils génétiques humains.

Ainsi aucun additif, aucun réactif ou aucune étape supplémentaire n'est à rajouter entre le prélèvement et l'amplification d'ADN avec les kits commerciaux (tels que les kits Identifiler plus, Identifiler direct, GlobalFiler et GlobalFiler express de la société Thermofisher).

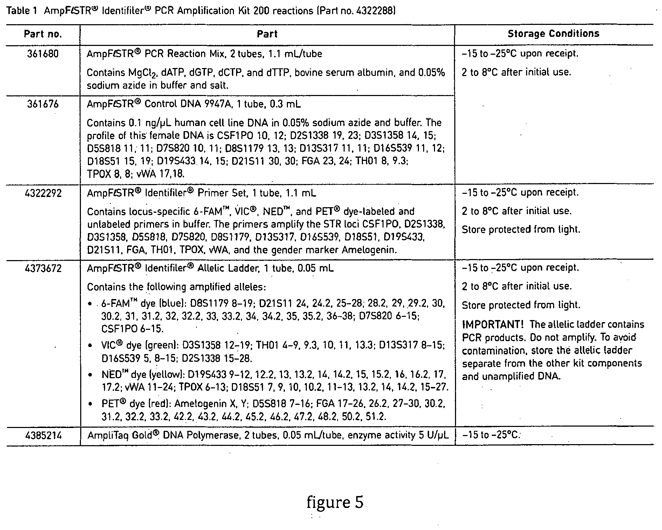

A titre d'exemple, la composition du kit Identifiler est telle que décrite dans la figure 5.

Ces kits peuvent donc être utilisés sans modification de leur composition initiale, en particulier sans ajouts de composants supplémentaires, ce qui est un gain très important en terme de coûts et de temps.

De plus, la faible superficie du corps absorbant permet non seulement de diminuer le volume réactionnel nécessaire d'amplification par rapport à celui préconisé par les fournisseurs, en conservant les mêmes proportions entre les différents réactifs générant ainsi des économies significatives mais permet également de limiter l'altération de la trace favorisant ainsi sa préservation pour des analyses ultérieures.

Le mélange de réaction d'amplification par PCR d'acides nucléiques tel qu'utilisé à l'étape 2) du procédé selon la présente invention fait référence à une combinaison particulière de réactifs qui sont nécessaires pour effectuer une réaction d'amplification par PCR. Le mélange de réaction d'amplification utilisé dans chaque cas et les conditions appliquées pour effectuer l'amplification sont bien connues de l'Homme du métier. Par exemple, le mélange de réaction d'amplification peut comprendre un ou plusieurs ensembles d'amorces pour veiller à ce qu'un acide nucléique cible particulier soit soumis à la réaction d'amplification, ainsi que des enzymes, des nucléotides, des sondes, des sels et des tampons qui peuvent également être nécessaires pour effectuer une amplification.

Ledit mélange de réaction d'amplification par PCR d'acides nucléiques est ajouté en quantité suffisante pour saturer le corps absorbant du dispositif selon l'invention. Il est typiquement ajouté en une quantité de 7, 5 à 25 microlitres, de préférence 12,5 à 15, de manière encore préférée 12,5 microlitres.

Ledit mélange de réaction de séquençage des acides nucléiques tel qu'utilisé à l'étape 3) du procédé selon la présente invention fait référence à une combinaison particulière de réactifs qui sont nécessaires pour effectuer un séquençage de fragments d'ADN amplifiés. Le mélange de réaction de séquençage utilisé dans chaque cas et les conditions appliquées pour réaliser le séquençage par la technique de l'électrophorèse sont bien connues de l'Homme du métier. Par exemple, le mélange de la réaction de séquençage peut comprendre de la formamide ainsi qu'un ensemble de fragments d'ADN de poids moléculaire connus, communément appelé standard de taille par l'Homme du métier. En fonction de la taille qui leur est attribuée, chaque fragment d'ADN amplifié est identifié par comparaison avec un ensemble de fragments d'ADN de référence communément appelé échelle allélique par l'Homme du métier.

Selon un mode de réalisation possible de l'invention, l'étape d'analyse peut être réalisée de manière directe ou indirecte. En particulier, pour une analyse indirecte, si le dispositif selon la présente invention comprend un corps absorbant composé de fibres synthétiques, de préférence en Nylon, floquées, le matériel biologique prélevé à l'étape 1) peut être libéré par mise en contact avec un volume d'eau stérile de 5 à 20 microlitres, de préférence 5 à 10 microlitres, puis un volume de ladite eau comprenant les acides nucléiques ainsi libérés du corps absorbant sont prélevés pour la réalisation de l'étape 2) d'amplification. De préférence le volume ainsi prélevé est de 5 microlitres.

Le procédé de collecte et d'analyse et/ou d'identification des acides nucléiques d'un matériel biologique mettant en oeuvre le dispositif selon l'invention ne nécessite que très peu d'étapes par rapports aux procédés classiquement utilisés pour de telles analyses et/ou identification, ce qui permet de le réaliser au plus près du lieux de collecte, dans un « laboratoire mobile », tel que par exemple un véhicule de type camionnette, dans lequel on pourra trouver au moins deux récipients de type plaque 96 puits, un milieu de réaction d'amplification par PCR d'acides nucléiques, un thermocycleur, un mélange de réaction de séquençage et un séquenceur.

De par sa très faible surface de contact et ses propriétés décrites ci-avant, le dispositif selon l'invention peut avantageusement être utilisé avec un dispositif miniature de détection biologique et/ou d'analyse génétique miniaturisé de type Lab-on-chip utilisant préférentiellement une technique microfluidique.

Les dessins annexés illustrent l'invention :

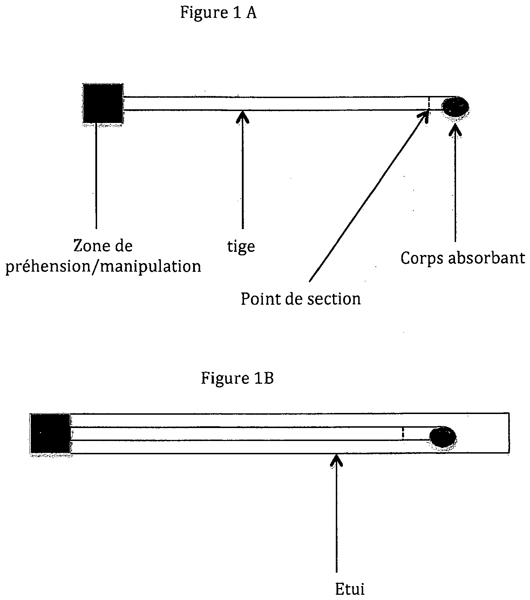

La figure 1 A représente en coupe, le dispositif de collection de l'invention, hors de son étui. La zone de préhension et de manipulation se situe à une extrémité et le corps absorbant à une extrémité opposée.
La figure 1 B représente le dispositif de collection dans son étui, avant ou après collection du matériel biologique avec un corps absorbant dans le prolongement rectiligne de la tige.

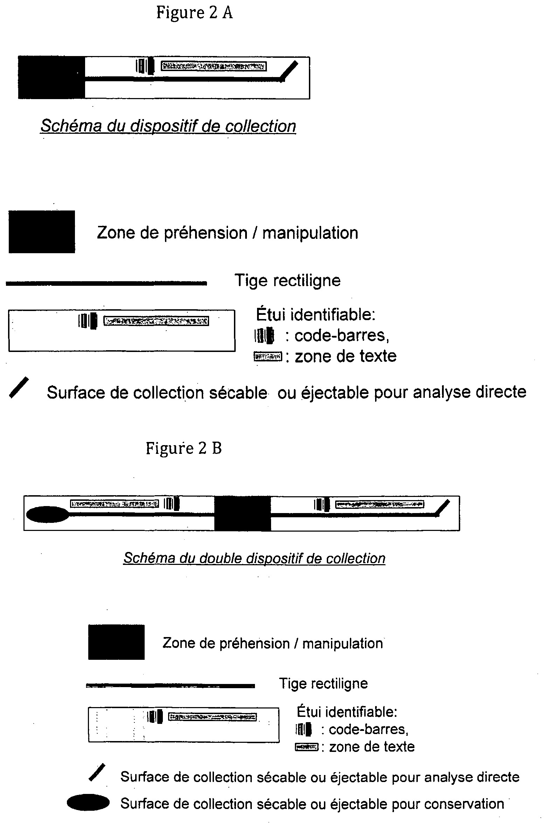

La figure 2 représente le dispositif de collection avec un corps absorbant (indiqué comme « surface de collection ») présentant un angle par rapport à la tige rectiligne permettant de faciliter la collecte, ainsi que son étui hermétique identifiable par code-barres.
La figure 3 représente une variante du dispositif de collection ou un second dispositif de collection présentant un second corps absorbant (appelé surface de collection pour conservation) est couplé au premier dispositif tel que présenté à la figure 2. La zone de préhension se trouve alors au milieu de la tige et non en son extrémité.

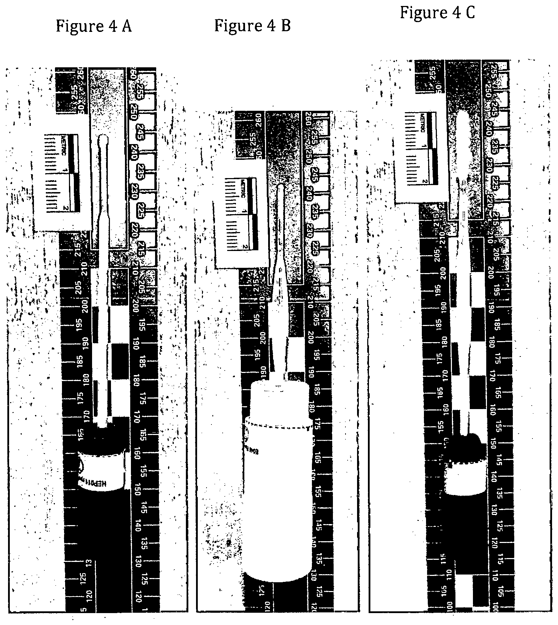

La figure 4A est une photographie du dispositif avec un corps absorbant d'une surface de 3,14 mm2, composé de fibres de nylon floquées sur la tige.
La figure 4B est une photographie du dispositif avec un corps absorbant d'une surface de 1,13 mm2, composé de fibres de nylon floquées sur la tige.
La figure 4C est une photographie d'un écouvillon standard FloqSwab 4N6 Crime Scene Cat 3509C, Regular tip in plain tube commercialisé par la société Copan La figure 5 est la composition du Kit IdentiFiler tel que commercialisé en 2015
La figure 6A est la comparaison des intensités moyennes de fluorescence obtenues à partir de 1 microlitre de sang collecté à l'aide d'un disque de cellulose (à gauche) et d'un écouvillon standard (à droite)
La figure 6B est la comparaison des intensités moyennes de fluorescence obtenues à partir de 5 microlitre de sang collecté à l'aide d'un disque de cellulose (à gauche) et de fibres synthétiques floquées sur une tige (à droite)
La figure 6C est la comparaison des intensités moyennes de fluorescence obtenues à partir de 1 microlitre de sperme collecté à l'aide d'un disque de cellulose (à gauche) et de fibres synthétiques floquées sur une tige (à droite)
La figure 7 est une photographie de 2 gouttes de sang de 5 microlitres prélevées avec le dispositif selon l'invention (surface 1,13 mm2)
La figure 8 est une empreinte digitale de sang prélevée avec un écouvillon standard et avec le dispositif selon l'invention (surface 1,13 mm2)
La figure 9 photographie d'une trace de sang d'un microlitre présente dans le canon d'une arme à feu et prélevée avec le dispositif selon l'invention (surface 1,13 mm2)

D'autres caractéristiques et avantages de l'invention pourront apparaître dans les exemples qui suivent, et sont donnés à titre purement illustratif et nullement limitatif.

### EXEMPLES

### Exemple 1 : détermination des surfaces optimales - matrice de cellulose

Afin de tester l'effet de la taille sur l'efficacité de la collecte, analyse et identification d'ADN, il a été procédé au test suivant :
Un corps absorbant solide et sec constitué d'une matrice de cellulose sèche comprenant des agents surfactants et dénaturant de type FTA (société GE) ou NucleiCard (société COPAN), sous la forme d'un disque (appelé « pastille biologique ») de diamètre de 1,2 mm (soit une surface de 1,13 mm2), 2mm (soit une surface de 3,14 mm2), 3 mm (soit une surface de 7,06 mm2) et un carré de 3,3 cm de côté (soit une surface de 10 mm2) ont été testés.

A défaut de disposer d'un dispositif de collecte industriel, objet de la présente invention, à savoir ledit corps absorbant fixé à une tige, la pastille biologique est manipulée à l'aide d'une pince de type pince brucelles. Il est noté par les utilisateurs une difficulté d'utilisation ne permettant pas d'imaginer une utilisation ainsi, avec de telles pinces au quotidien, notamment par les forces de police sur les scènes d'infraction. La méthode de collecte expérimentale se décompose de la manière suivante : collecte de la trace biologique par essuyage du support à l'aide de mouvements de balayages horizontaux, circulaires ou par simples tamponnages en fonction de la nature de la surface à essuyer.

Les tests on été effectués sur différents types de traces susceptibles de présenter de l'ADN en quantité et qualité variables mais principalement dégradé. A cet effet, les collectes ont été effectuées sur des traces dites « de contact » (essuyage de différents supports comme l'intérieur d'un véhicule ou objets du quotidien), des traces de sang, des cellules buccales et des tissus biologiques divers (éléments de corps dans des états de dégradation avancés tel que la putréfaction ou la carbonisation) avec à chaque fois un échantillon de 1 microlitre. Le corps absorbant est utilisé sec, sans humidification de la trace ou dudit corps absorbant avant collecte.

Après collecte, le corps absorbant est mis en contact avec un milieu de réaction d'amplification de fragments d'ADN de type STR (Short Tandem Repeat) tels que les kits Identifiler plus et Globalfiler (Lifetechnologies) puis inséré dans le thermocycleur pour PCR.

Ainsi, sans délai, à l'issue de la collecte, l'ADN piégé dans la pastille biologique est directement amplifié par PCR (Polymerase Chain Reaction) dans un thermocycleur (GeneAmp PCR system 9700), sans qu'il soit nécessaire d'ajouter un additif pour stabiliser la réaction d'amplification, aux fins de détermination de profils génétiques humains. Cette réaction d'amplification est effectuée à l'aide de kits commerciaux d'amplification de fragments d'ADN de type STR (Short Tandem Repeat) tels que les kits Identifiler plus et Globalfiler (Lifetechnologies) selon les recommandations préconisées par le fournisseur. Ainsi dans le cas d'une utilisation d'Identifiler plus, le thermocycleur est utilisé avec 28 cycles de PCR et avec Globalfiler avec 29 cycles de PCR.

Le génotypage des produits de PCR est ensuite effectué à l'aide de l'analyseur ABI 3500 xl (Lifetechnologies). L'analyse des résultats est réalisée à l'aide du logiciel GeneMapper ID-X version 1.2.

Les résultats obtenus montrent que les surfaces de 10 mm2 et de 7,06 mm2 ne permettent pas d'identifier le profil ADN de l'échantillon, concordant avec l'idée généralement admise par l'Homme du métier qu'un échantillon d'ADN sous forme de microtrace de l'ordre de 1 microlitre ne peut pas être collecté et analysé avec de tels dispositifs de collecte.

Toutefois, et c'est là une partie de la solution technique objet de la présente invention, la Demanderesse a découvert de manière surprenante et inattendue, qu'en diminuant encore la surface de la pastille biologique, en réalisant les mêmes tests avec des surfaces de corps absorbant plus petite, de 1,13 et 3,14 mm2 il était possible d'obtenir un profil ADN partiel (50% des allèles identifiés) dans le cas de la surface de 3,14 mm2 et même un profil ADN complet (100% des allèles identifiés) avec la surface de 1,13 mm2, allant à l'encontre du préjugé technique de l'Homme du métier. Ainsi la Demanderesse a démontré qu'il était non seulement possible de collecter mais également d'analyser et identifier avec précision une microtrace d'ADN de l'ordre d'1 microlitre, à l'aide d'un dispositif de collecte.

Afin de valider l'efficacité du processus d'analyse rapide, les traces biologiques ont également été collectées à l'aide d'un dispositif conventionnel de type écouvillon (FloqSwab, société COPAN) dans des quantités de 1, 3 et 5 microlitres, puis analysées à l'aide des kits Identifiler Plus, Identifiler Direct et GlobalFiler selon les recommandations du fournisseur sans quantification au préalable de l'ADN extrait de l'intégralité de l'extrémité de l'écouvillon.

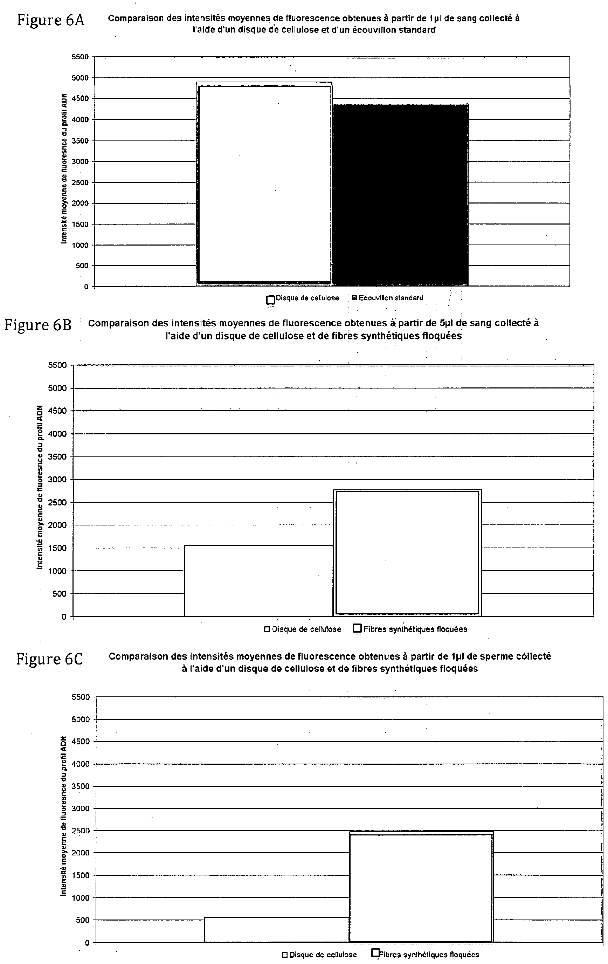

Les résultats obtenus à partir du processus d'analyse rapide, objet de la présente invention, sont meilleurs que ceux obtenus à l'aide du processus d'analyse conventionnel, avec étape de lyse cellulaire et d'extraction obligatoire (figure 6A). En effet, le système de collection et d'analyse rapide est principalement plus performant sur les traces biologiques de très petites superficies et volumes (microtraces de l'ordre de 1mm² et 1µl) en raison de la miniaturisation de la surface de collection et l'absence de perte de matériel génétique prélevé (en raison de l'absence d'extraction d'ADN à partir du dispositif de collecte).

### Exemple 2 : détermination de la composition optimale du corps absorbant -disque de cellulose / fibres synthétiques floquées - surface 1,13 mm2

Des tests comparatifs d'efficacité sont effectués entre un corps absorbant composé d'un disque de cellulose de 1,13 mm2 comprenant des agents dénaturants et surfactants et des fibres synthétiques d'une surface totale de 1,13 mm2 floquées sur une tige et comprenant ces mêmes agents dénaturants et surfactants.

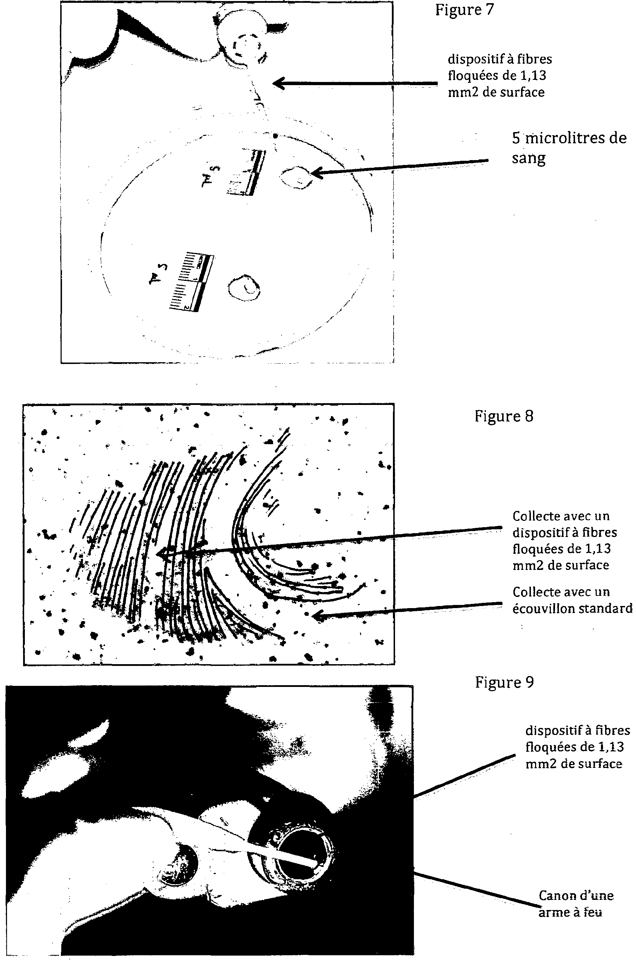

Deux types de traces sont collectées :
- une trace de sang à partir de 5µl de sang sur une lame de verre (figure 7)
- une trace de sperme à partir de 1µl de sperme séché sur une lame de verre.
Directement à l'issue de la collecte, les corps absorbants sont déposés dans les puits d'une microplaque 96 puits contenant 25µl de mélange de réaction d'amplification de fragments d'ADN de type STR (Short Tandem Repeat) fourni avec le kit GlobalFiler (Lifetechnologies), puis la microplaque est insérée dans un thermocycleur (Gene Amp PCR system 9700) pour la réaction de PCR. Le génotypage des produits de PCR est ensuite effectué à l'aide de l'analyseur séquenceur ABI 3500 XL (lifetechnologies). L'analyse des réactifs est effectuée à l'aide du logiciel GeneMapper ID-X version 1.2.

Les résultats permettent de mettre en évidence des profils ADN identiques avec la cellulose et avec les fibres synthétiques floquées, à savoir une identification de 100% du profil ADN pour les prélèvements de sang et de sperme. En revanche, le dispositif de fibres synthétiques floquées s'avère plus efficace que le dispositif du disque de cellulose car l'intensité de fluorescence moyenne du profil génétique obtenu avec les fibres floquées est supérieure à celle obtenue avec le disque de cellulose (figure 6 B et 6 C).

En outre, un vrai confort d'utilisation a été noté avec le dispositif dans sa version de fibres synthétiques floquées en comparaison avec le dispositif du disque de cellulose manipulé à l'aide de pinces brucelles d'autant plus lorsque la trace à collecter est difficile d'accès comme cela peut être le cas à l'intérieur du canon d'une arme à feu (figure 9).

### Exemple 3 : vérification des performances du dispositif de fibres synthétiques floquées de 1,13 mm2 et traitées avec des agents dénaturants et surfactants par rapport à un écouvillon standard (4N6 FloqSwab, commercialisé par la société COPAN)

Des tests comparatifs d'efficacité sont effectués entre des fibres synthétiques d'une surface totale de 1,13 mm2 floquées sur une tige et comprenant des agents dénaturants et surfactants.

Deux types de traces sont collectées :
1. une empreinte de pouce révélée sur une vitre
2. une empreinte de pouce ensanglantée révélée sur le sol (carrelage)
Dans le cas 1. la partie supérieure de l'empreinte de pouce est collectée avec le dispositif de fibres synthétiques floquées alors que la partie inférieure de l'empreinte de pouce est collectée avec un écouvillon standard.
Dans le cas 2. un essuyage au moyen des fibres synthétiques floquées de 1,13 mm2 a permis de collecter seulement le matériel biologique matérialisé par deux crêtes du dessin papillaire en raison de la faible superficie du dispositif. En revanche, 5 crêtes ont été collectées avec l'écouvillon standard altérant d'autant plus le dessin papillaire. Directement à l'issue de la collecte, les corps absorbants sont déposés dans les puits d'une microplaque 96 puits contenant 25µl de mélange de réaction d'amplification de fragments d'ADN de type STR (Short Tandem Repeat) fourni avec le kit GlobalFiler (Lifetechnologies), puis la microplaque est insérée dans un thermocycleur (Gene Amp PCR system 9700) pour la réaction de PCR. Le génotypage des produits de PCR est ensuite effectué à l'aide de l'analyseur séquenceur ABI 3500 XL (lifetechnologies). L'analyse des réactifs est effectuée à l'aide du logiciel GeneMapper ID-X version 1.2.
Le prélèvement effectué au moyen du processus d'analyse rapide à partir de fibres synthétiques floquées de 1,13 mm² sur la trace papillaire révélée sur la vitre a permis d'obtenir une meilleur résultat que celui obtenu à l'aide du processus d'analyse conventionnel avec étape de lyse cellulaire et d'extraction obligatoire. En effet, un profil ADN complet a été obtenu à partir du processus d'analyse rapide alors qu'un profil ADN très partiel a été obtenu à partir du processus d'analyse standard.
Les prélèvements effectués sur l'empreinte de pouce ensanglantée ont permis d'obtenir un profil ADN complet à des intensités moyennes de fluorescences équivalentes entre les deux dispositifs de collecte et d'analyse. En revanche, la possibilité de prélever seulement deux crêtes du dessin papillaire à l'aide du dispositif de collection et d'analyse rapide au lieu de 4 à 5 crêtes à l'aide du dispositif de collection et d'analyse pour un résultat équivalent démontre toute la sensibilité et l'intérêt du dispositif de collection et d'analyse rapide pour limiter l'altération de la trace (figure 8).

## Revendications

1. Dispositif de collection de matériel biologique, **caractérisé en ce qu'**il comprend une tige présentant à au moins une extrémité un corps absorbant sec sous forme de fibres synthétiques floquées d'une surface comprise entre 1 et 2 mm2, ledit corps absorbant comprenant des agents surfactants et dénaturants.

2. Dispositif selon la revendication 1, **caractérisé en ce que** ladite extrémité présentant ledit corps absorbant est sécable ou éjectable.

3. Dispositif selon l'une quelconque des revendications 1 à 2 **caractérisé en ce que** ledit matériel biologique est sélectionné dans le groupe consistant en des fluides corporels physiologiques, des cellules ou suspensions cellulaires d'humains ou d'animaux, en particulier une trace de contact, du sang, de la moelle osseuse, des cellules buccales, du sperme, de la salive, du liquide cervical, des cheveux, des poils, de la peau, du muscle, du sérum, du fluide synovial, du fluide spinal cérébral, du fluide lymphatique, des cellules vaginales, de l'urine, des fèces, et plus largement tout tissu biologique humain ou animal dégradé ou non ; des fluides corporels physiologiques ou des suspensions cellulaires de plantes ; des produits liquides, extraits ou suspensions de bactéries, de champignons, de plasmides, de parasites, ou de virus ; des extraits liquides ou des homogénats de tissus corporels humains ou animaux ; des milieux issus d'une synthèse d'acides nucléiques ; et des mélanges d'acides nucléiques synthétisés chimiquement ou biochimiquement.

4. Dispositif selon l'une quelconque des revendications 1 à 3 **caractérisé en ce qu'**il comprend un étui hermétique.

5. Kit pour la collecte, l'analyse et/ou l'identification de matériel biologique comprenant un dispositif selon la revendication 4, deux récipients, au moins un mélange de réaction d'amplification par PCR d'acides nucléiques, un thermocycleur, un mélange de réaction de séquençage et un séquenceur.

6. Laboratoire mobile pour la collecte, l'analyse et/ou l'identification de matériel biologique comprenant un kit selon la revendication 5 dans un véhicule mobile motorisé.

7. Utilisation d'un dispositif selon l'une quelconque des revendications 1 à 4 pour le prélèvement de matériel biologique à partir d'une trace biologique présentant toute surface à partir d'une micro-surface de l'ordre de 1 mm2 ou présentant tout volume, à partir d'un micro-volume de l'ordre de 1 microlitre.

8. Utilisation d'un dispositif selon l'une quelconque des revendications 1 à 4 pour le prélèvement de matériel biologique **caractérisé en ce que** si le prélèvement est effectué avec le dispositif directement sur un être humain, ledit être humain n'est pas vivant.

9. Procédé de collecte de matériel biologique, **caractérisé en ce qu'**il comprend une étape de mise en contact du dispositif selon l'une quelconque des revendications 1 à 4 avec ledit matériel biologique.

10. Procédé de collecte de matériel biologique selon la revendication 9 **caractérisé en ce que** lorsque le matériel biologique à collecter est humide, la collecte est effectuée directement, et lorsque le matériel biologique est sous forme sèche, ledit procédé comprend une étape préalable d'humidification dudit corps absorbant du dispositif ou d'humidification dudit matériel biologique, avec de l'eau stérile et/ou apyrogène.

11. Procédé de collecte et d'analyse et/ou d'identification des acides nucléiques d'un matériel biologique, **caractérisé en ce qu'**il comprend :
1. la ou les étapes du procédé de collecte dudit matériel biologique selon les revendications 9 ou 10 ;
2. une étape d'amplification des acides nucléiques, de préférence de l'ADN, dudit matériel biologique collecté sur le corps absorbant du dispositif comprenant :
∘ la mise en contact dudit corps absorbant comprenant le matériel biologique collecté avec au moins un mélange de réaction d'amplification par PCR d'acides nucléiques ;
∘ la réaction d'amplification par PCR ;
3. une étape de séquençage des acides nucléiques amplifiés à l'étape 2 comprenant la mise en contact desdits acides nucléiques avec un mélange de réaction de séquençage, le séquençage et l'analyse du résultat de séquençage.

## Patentansprüche

1. Vorrichtung zum Sammeln von biologischem Material,
**dadurch gekennzeichnet, dass** sie einen Stab umfasst, der an mindestens einem Ende einen trockenen Absorptionskörper in Form von beflockten Kunstfasern mit einer Oberfläche zwischen 1 und 2 mm2 aufweist, der Absorptionskörper umfassend Tenside und Denaturierungsmittel.

2. Vorrichtung nach Anspruch 1, **dadurch gekennzeichnet, dass** das Ende, das den Absorptionskörper aufweist, abtrennbar oder ausstoßbar ist.

3. Vorrichtung nach einem der Ansprüche 1 bis 2,
**dadurch gekennzeichnet, dass** das biologische Material aus der Gruppe ausgewählt ist, bestehend aus physiologischen Körperfluiden, Zellen oder Zellsuspensionen von Menschen oder Tieren, insbesondere eine Kontaktspur, Blut, Knochenmark, bukkalen Zellen, Sperma, Speichel, Zervixflüssigkeit, Haaren, Körperhaaren, Haut, Muskel, Serum, Synovialfluid, Gehirnrückenmarkfluid, Lymphfluid, Vaginalzellen, Urin, Fäkalien und im weiteren Sinne jeglichem abgebauten oder nicht abgebauten menschlichen oder tierischen biologischen Gewebe; physiologischen Körperfluiden oder Pflanzenzellsuspensionen; flüssigen Produkten, Extrakten oder Suspensionen von Bakterien, Pilzen, Plasmiden, Parasiten oder Viren; flüssigen Extrakten oder Homogenaten von menschlichen oder tierischen Körpergeweben; Medien, die aus einer Nukleinsäuresynthese stammen; und Mischungen von chemisch oder biochemisch synthetisierten Nukleinsäuren.

4. Vorrichtung nach einem der Ansprüche 1 bis 3,
**dadurch gekennzeichnet, dass** sie eine hermetische Hülle umfasst.

5. Kit zum Sammeln, Analysieren und Identifizieren von biologischem Material, umfassend die Vorrichtung nach Anspruch 4, zwei Behälter, mindestens eine Reaktionsmischung einer Nukleinsäureamplifikation durch PCR, einen Thermocycler, eine Reaktionsmischung einer Sequenzierung und einen Sequenzieren

6. Mobiles Labor zum Sammeln, Analysieren und Identifizieren von biologischem Material, umfassend das Kit nach Anspruch 5 in einem motorisierten mobilen Fahrzeug.

7. Verwendung einer Vorrichtung nach einem der Ansprüche 1 bis 4 für die Probeentnahme von biologischem Material ausgehend von einer biologischen Spur, die jegliche Oberfläche aufweist, ausgehend von einer Mikrooberfläche in der Größenordnung von 1 mm2, oder die jegliches Volumen aufweist, ausgehend von einem Mikrovolumen in der Größenordnung von 1 Mikroliter.

8. Verwendung einer Vorrichtung nach einem der Ansprüche 1 bis 4 für die Probeentnahme von biologischem Material, **dadurch gekennzeichnet, dass,** falls die Probeentnahme mit der Vorrichtung direkt an einem menschlichen Wesen vorgenommen wird, das menschliche Wesen nicht lebend ist.

9. Verfahren zum Sammeln von biologischen Material,
**dadurch gekennzeichnet, dass** es einen Schritt eines Inberührungbringens der Vorrichtung nach einem der Ansprüche 1 bis 4 mit dem biologischen Material umfasst.

10. Verfahren zum Sammeln von biologischem Material nach Anspruch 9, **dadurch gekennzeichnet, dass,** wenn das zu sammelnde biologische Material feucht ist, das Sammeln direkt vorgenommen wird, und wenn das biologische Material in trockener Form ist, das Verfahren einen vorangehenden Schritt einer Befeuchtung des Absorptionskörpers der Vorrichtung oder einer Befeuchtung des biologischen Materials mit sterilisiertem und/oder pyrogenfreiem Wasser umfasst.

11. Verfahren zum Sammeln und Analysieren und/oder Identifizieren von Nukleinsäuren eines biologischen Materials, **dadurch gekennzeichnet, dass** es umfasst:
1. den oder die Verfahrensschritte des Sammelns des biologischen Materials nach den Ansprüchen 9 oder 10;
2. einen Schritt eines Amplifizierens von Nukleinsäuren, vorzugsweise von DNA, des biologischen Materials, das auf dem Absorptionskörper der Vorrichtung gesammelt wird, umfassend:
o das Inberührungbringen des Absorptionskörpers, umfassend das gesammelte biologische Material, mit einer Reaktionsmischung der Nukleinsäureamplifikation durch PCR;
o die Amplifikationsreaktion durch PCR;
3. einen Schritt des Sequenzierens der Nukleinsäuren, die in Schritt 2 amplifiziert werden, umfassend das Inberührungbringen der Nukleinsäuren mit einer Sequenzierungsreaktionsmischung, das Sequenzieren und das Analysieren des Sequenzierungsergebnisses.

## Claims

1. Device for collecting biological material, **characterized in that** it comprises a rod having, at at least one end, a dry absorbent body in the form of flocked synthetic fibers having a surface of between 1 and 2 mm2, said absorbent body comprising surfactants and denaturing agents.

2. Device according to claim 1, **characterized in that** said end having said absorbent body is scored or ejectable.

3. Device according to any of claims 1 to 2, **characterized in that** said biological material is selected from the group consisting of physiological bodily fluids, human or animal cells or suspension cells, in particular a contact trace, blood, bone marrow, buccal cells, sperm, saliva, cervical liquid, hair, fur, skin, muscle, serum, synovial fluid, cerebral spinal fluid, lymphatic fluid, vaginal cells, urine, feces, and more broadly any degraded or non-degraded human or animal biological tissue; physiological bodily fluids or plant cell suspensions; liquid products, extracts or suspensions of bacteria, fungi, plasmids, parasites, or viruses; liquid extracts or homogenates of human or animal body tissue; media from a nucleic acid synthesis; and mixtures of chemically or biologically synthesized nucleic acids.

4. Device according to any of claims 1 to 3, **characterized in that** it comprises a sealed case.

5. Kit for collecting, analyzing and/or identifying biological material comprising a device according to claim 4, two containers, at least one nucleic acid PCR amplification reaction mixture, a thermocycler, a sequencing reaction mixture and a sequencer.

6. Mobile laboratory for collecting, analyzing and/or identifying biological material comprising a kit according to claim 5 in a motor-driven mobile vehicle.

7. Use of a device according to any of claims 1 to 4 for sampling biological material from a biological trace having any surface from a micro-surface in the range of 1 mm2 or having any volume, from a micro-volume in the range of 1 microliter.

8. Use of a device according to any of claims 1 to 4 for sampling biological material, **characterized in that** if the sample is taken with the device directly on a human being, said human being is not alive.

9. Method for collecting biological material, **characterized in that** it comprises a step of contacting the device according to any of claims 1 to 4 with said biological material.

10. Method for collecting biological material according to claim 9, **characterized in that** when the biological material to be collected is wet, the collection is carried out directly, and when the biological material is in dry form, said method comprises a prior step of humidifying said absorbent body of the device or humidifying said biological material with sterile and/or pyrogen-free water.

11. Method for collecting and analyzing and/or identifying nucleic acids of a biological material, **characterized in that** it comprises:
1. the step or steps of the method for collecting said biological material according to claims 9 or 10;
2. a step of amplifying nucleic acids, preferably DNA, of said biological material collected on the absorbent body of the device comprising:
o contacting said absorbent body comprising the collected biological material with at least one nucleic acid PCR amplification reaction mixture;
o the PCR amplification reaction;
3. a step of sequencing nucleic acids amplified in step 2 comprising contacting said nucleic acids with a sequencing reaction mixture, the sequencing and the analysis of the sequencing result.
